Europäisches Patentamt

European Patent Office

Office européen des brevets

(11) Veröffentlichungsnummer: **0 346 697**
**A2**

## (12) EUROPÄISCHE PATENTANMELDUNG

(21) Anmeldenummer: 89109953.3

(22) Anmeldetag: 01.06.89

(51) Int. Cl.⁴: **A61F 5/01 , A61F 13/00 ,**
**A61F 2/60**

(30) Priorität: 13.06.88 DE 3820098

(43) Veröffentlichungstag der Anmeldung:
20.12.89 Patentblatt 89/51

(84) Benannte Vertragsstaaten:
AT BE CH DE ES FR GB GR IT LI LU NL SE

(71) Anmelder: W.L. Gore & Associates GmbH
Hermann-Oberth-Strasse 22
D-8011 Putzbrunn(DE)

(72) Erfinder: Hübner, Thorger
Breitensteinstrasse 28
D-8208 Kolbermoor(DE)
Erfinder: Bleimhofer, Walter
Barbarastrasse 38
D-8122 Penzberg(DE)

(74) Vertreter: Klunker . Schmitt-Nilson . Hirsch
Winzererstrasse 106
D-8000 München 40(DE)

(54) Orthopädische Körperhilfsvorrichtung.

(57) Orthopädische Körperhilfsvorrichtungen insbesondere Körpergliedprothesen, Gipsschienen, Luftschienen oder dergleichen, sind mindestens in dem
auf der Haut des Patienten aufliegenden Bereich mit
einem Ventilationsschichtmaterial ausgekleidet, das
Längsluftleitfähigkeit in Längserstreckungsrichtung
und damit in Verbindung stehende Querluftleitfähigkeit zu mindestens einer seiner Oberflächen aufweist
und das derart druckstabil ist, daß die Längsluftleitfähigkeit bei normaler Benutzung der Körperhilfsvorrichtung erhalten bleibt.

Fig. 1

## Orthopädische Körperhilfsvorrichtung

Die Erfindung macht orthopädische Körperhilfsvorrichtungen mit einem flächigen, flexiblen, druckstabilen Ventilationsschichtmaterial gemäß Anspruch 1 verfügbar.

Man kommt häufig mit atmungsinaktiven oder atmungsschwachen Materialien direkt oder indirekt in Berührung, was an der entsprechenden Körperstelle zur Bildung von Schweißfeuchtigkeit führt.

Als erstes Beispiel wird eine Körpergliedprothese, beispielsweise Beinprothese, betrachtet. Solche Prothesen bestehen im allgemeinen aus einem Kunststoffmaterial oder Holz. An einer Aufstandsfläche kommt die Haut des Prothesenträgers mit dem Kunststoffmaterial oder Holz der Prothese in Berührung. Im Fall einer Beinprothese ist dies ein Teil des Beinstumpfes, an dem die Beinprothese befestigt wird. Das Holz oder Kunststoffmaterial der Prothese ist glatt und luftundurchlässig. Insbesondere bei warmer Außentemperatur kommt es daher zu Schweißbildung an der Aufstandsfläche. Dies ist für den Prothesenträger unangenehm und kann auch zu beträchtlichen Hautreizungen führen.

Als zweites Beispiel seien Gipsschienen betrachtet, beispielsweise in Schalenform. Der zu schienende Körperteil, beispielsweise ein Unterarm, wird mit der Gipsschiene oder der Gipsschale geschient. Dabei ist die Haut des Patienten praktisch mit der gesamten Schienenoberfläche der Gipsschiene oder Gipsschale in Berührung. Auch hier kommt es an der Berührungsfläche zwischen Haut und Gipsschiene bzw. Gipsschale wieder zur Bildung von Körperfeuchtigkeit, die nicht oder kaum entweichen kann. Dies macht das Tragen solcher Gipsschienen oder Gipsschalen unangenehm und führt häufig zu Hautreizen wie Hautjukken.

Ein weiteres Beispiel sind aufblasbare Luftschienen, die um den zu schienenden Körperteil herumgelegt und dann aufgeblasen werden. Derartige Luftschienen weisen im allgemeinen eine Kunststoffhaut auf, die an der Haut des zu schienenden Körperteils anliegt. Hierbei besteht im allgemeinen eine besonders innige Verbindung zwischen der Körperhaut des Patienten und der Außenhaut der Luftschiene, so daß die Bildung und Ansammlung von Schwitzfeuchtigkeit besonders stark sind.

Dagegen kann Abhilfe durch mindestens teilweise Auskleidung der orthopädischen Körperhilfseinrichtung mit dem erfindungsgemäßen Ventilationsschichtmaterial geschaffen werden. Dadurch, daß dieses Ventilationsschichtmaterial in Längsrichtung leitende Luftkanäle und damit in Verbindung stehende und in Querrichtung bis zu mindestens einer seiner Oberflächen reichende Luftkanäle aufweist, kann Luft an alle mit dem Ventilationsschichtmaterial ausgekleideten Bereich herankommen. Dadurch kann Schwitzfeuchtigkeit verdunsten und abgeführt werden. Die Ventilationswirkung wird im allgemeinen dadurch erhöht, daß der mit dem Ventilationsschichtmaterial versehene Gegenstand, sei es nun eine Prothese, eine Gipsschiene, eine Gipsschale, eine Luftschiene oder Ähnliches, während seiner Benutzung häufig bewegt wird, was in dem Ventilationsschichtmaterial zu einer Pumpwirkung führt, durch welche das Ventilations verhalten erhöht wird.

Die Ausrichtung des Ventilationsschichtmaterials in der orthopädischen Körperhilfseinrichtung ist zweckmäßigerweise derart, daß die Längsluftkanäle zu einem offenen Ende der Körperhilfseinrichtung gerichtet sind, während die Querluftleitkanäle zur Körperoberfläche des Patienten hin gerichtet sind.

Das Ventilationsschichtmaterial kann gänzlich verschieden strukturiert sein, beispielsweise offenporig, rippenartig, genoppt, mit Stegen versehen, netzartig dreidimensional oder dergleichen. Wichtig ist nur, daß dabei Längsluftleitkanäle und mit diesen in Verbindung stehende Querluftleitkanäle bis zu mindestens einer Oberfläche hin entstehen.

Neben der Ventilationswirkung hat das erfindungsgemäß verwendete Ventilationsschichtmaterial noch weitere Vorteile. Ein solcher Vorteil ist die Polsterwirkung, die beispielsweise im Fall von Gipsschienen sehr angenehm sein kann.

Das Ventilationsschichtmaterial soll in gewissem Umfang druckstabil sein. D.h., es soll bei der Druckbelastung, die bei normaler Verwendung der mit dem Ventilationsschichtmaterial versehenen Körperhilfsvorrichtung auftritt, seine Ventilationsfunktion nicht verlieren. D.h., bei einem solchen Normalbelastungsdruck sollen die Luftkanäle noch soweit ihre Form bewahren, daß sie noch ausreichend Ventilationsluft leiten können.

Das Ventilationsschichtmaterial kann aus geeignetem Kunststoff hergestellt sein. Beispiele hierfür sind Polyamid, Polypropylen und Polyester, in Monofilament-oder in Multifilamentform, gewebt oder geraschelt, oder auch in Form von retikuliertem Schaumstoff aus derartigem Material.

Eine vorteilhafte Weiterbildung der Erfindung besteht darin, an die Längsluftleitkanäle des Ventilationsschichtmaterials eine Ventilationsluftpumpe anzuschliessen, die entweder elektrisch betrieben wird oder durch mechanischen Antrieb unter Ausnutzung einer Körperbewegung des Patienten, im Fall einer Beinprothese der Kniebewegung, wobei z. B. bei der Knieknickbewegung Druck auf einen Blasebalg ausgeübt wird.

Das Ventilationsschichtmaterial, gegebenenfalls zusammen mit einer Innenauskleidung des Ventilationsmaterials, wird vorzugsweise als aus der Prothese herausnehmbarer Einsatz ausgebildet. Dies erleichtert das Waschen dieser Materialien

Vorteilhafte Weiterbildungen der Erfindung sind in den Ansprüchen 2 bis 10 angegeben. Gegenstände, bei denen die Verwendung von Ventilationsschichtmaterial besonders geeignet und vorteilhaft ist, sind in den Ansprüchen 2 bis 11 angegeben.

Die Erfindung wird nun anhand von Ausführungsformen näher erläutert. In den Zeichnungen zeigen:

Fig. 1 eine Ausführungsform eines erfindungsgemäßen Ventilationsschichtmaterials;

Fig. 2 in schematischer Darstellung einen Ausschnitt einer Körperprothese, die mit erfindungsgemäßem Ventilationsschichtmaterial versehen ist;

Fig. 3 in schematischer Darstellung einen Teil einer Beinprothese mit einem Schichtenaufbau gemäß Fig. 2; und

Fig. 4 in schematischer Darstellung eine aufblasbare Luftschiene mit erfindungsgemäßem Ventilationsschichtmaterial.

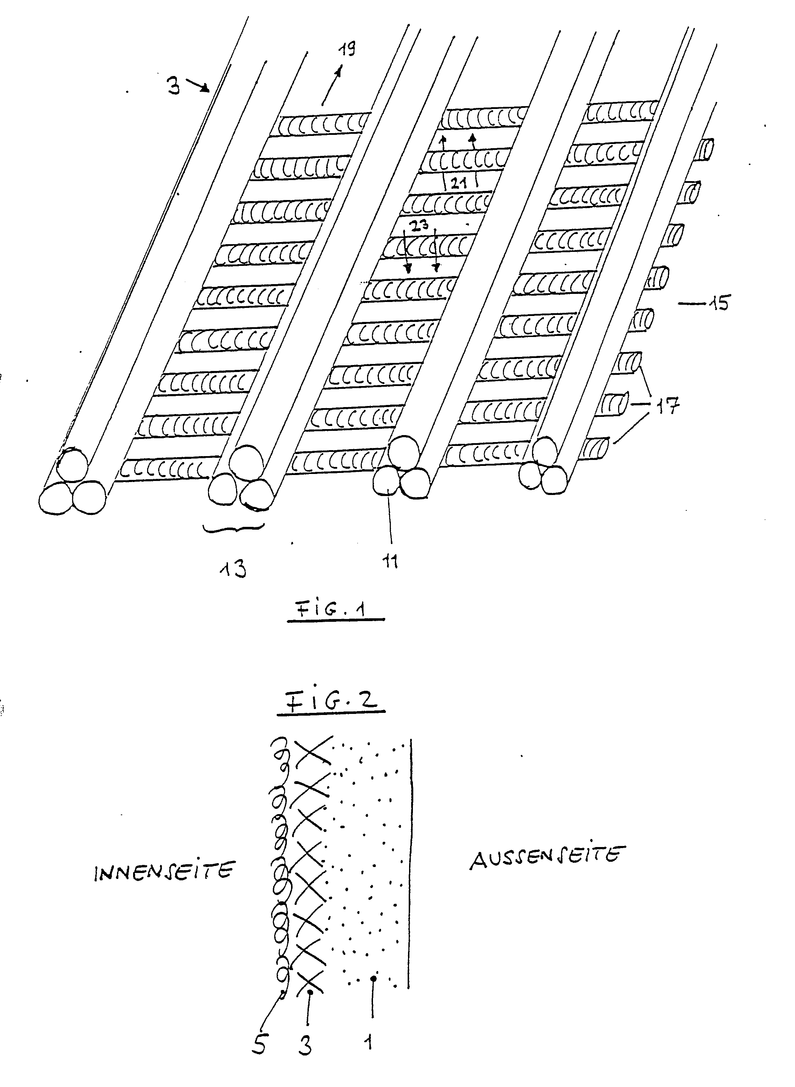

In Fig. 1 ist in schematischer, stark vergrößerter Weise ein Ausführungsbeispiel für ein erfindungsgemäß verwendbares Ventilationsschichtmaterial dargestellt. Dabei bilden je drei deltaförmig gebündelte Längsstege l3 einen Abstandshalter 11. Eine Vielzahl im wesentlichen parallel und mit Abstand voneinander angeordneter Abstandshalter 11 ist auf ihrer Unterseite mittels einer Vielzahl von bezüglich der Abstandshalter 11 quer verlaufenden, im Abstand voneinander angeordneten und im wesentlichen parallel zueinander verlaufenden Querstegen 17 verbunden. Die Zwischenräume zwischen je zwei benachbarten Abstandshaltern 11 bilden Längsluftleitkanäle 19. Damit in Verbindung stehen Querluftleitkanäle 21, 23, wobei die Querluftleitkanäle 23 ebenfalls durch die Zwischenräume zwischen den Abstandshaltern 11 gebildet sind und durch die Längsluftleitkanäle 19 strömende Luft zu der in Fig. 1 oberhalb der Abstandshalter 11 befindlichen Ebene leiten. Die Querluftleitkanäle 21 sind durch die Zwischenräume zwischen den Querstegen 17 gebildet und leiten durch die Längsluftleitkanäle 19 strömende Luft zu der in Fig. 1 unteren Seite des Ventilationsschichtmaterials. Feuchtigkeit, die sich unterhalb oder oberhalb des Ventilationsschichtmaterials bildet, kann über die Querluftleitkanäle 21 oder 23 und die Längsluftleitkanäle 19 abgeführt werden.

Fig. 2 zeigt in schematischer Darstellung einen Ausschnitt aus dem Querschnitt einer Körperprothese. Diese weist auf der Außenseite eine glatte Holz- oder Kunststoffschicht auf. Deren Innenseite ist mit Ventilationsschichtmaterial 3 ausgekleidet. Auf deren Innenseite befindet sich eine Auskleidungsschicht 5. Wenn das Ventilationsschichtmaterial 3 auf seiner inneren Oberfläche genügend glatt und hautverträglich ist, kann die innere Auskleidung 5 weggelassen werden. Wird dagegen ein Ventilationsschichtmaterial 3 mit einer Struktur verwendet, die auf der Innenoberfläche zu einer zu starken Rauhheit für die Haut führt, kann eine glatte, hautverträgliche innere Auskleidungsschicht 5 verwendet werden, die durch Perforation oder andere Maßnahmen eine genügend hohe Luftdurchlässigkeit aufweist, um die Ventilationswirkung des Ventilationsschichtmaterials 3 nicht zu beeinträchtigen.

Vorzugsweise sind das Ventilationsschichtmaterial 3 und gegebenenfalls die Auskleidungsschicht 5 als aus der Prothese herausnehmbarer Einsatz ausgebildet, der dann leichter gewaschen werden kann.

Im Fall einer Körperprothese wird es sich bei der Außenschicht 1 im allgemeinen um Holz oder einen Kunststoff handeln. Im Fall einer erfindungsgemäßen Gipsschiene handelt es sich bei der Außenschicht 1 um Gips, auf dessen Innenseite das Ventilationsschichtmaterial 3 vorgesehen ist und gegebenenfalls die Innenauskleidung 5.

Für den Fall einer erfindungsgemäßen Luftschiene, die nach Art von aufblasbaren Schwimmflügeln durch eine durch zwei Kunststoffhäute begrenzte aufblasbare Luftkammer gebildet wird, handelt es sich bei der Außenschicht 1 um die innere Kunststofflage der Luftkammer, auf deren Innenseite Ventilationsschichtmaterial 3 und gegebenenfalls die innere Auskleidung 5 angeordnet ist.

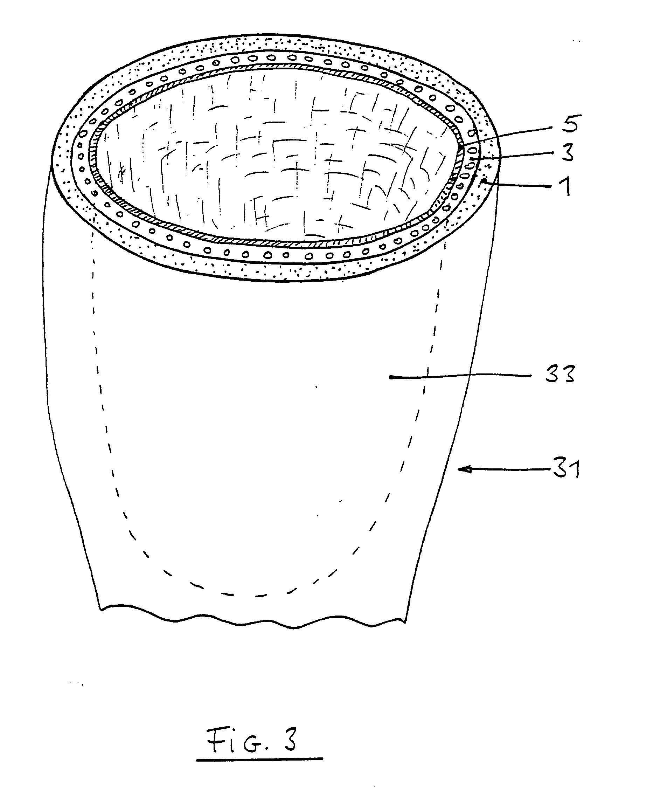

In Fig. 3 ist als ein Ausführungsbeispiel der Erfindung in schematischer Darstellung ein Ausschnitt einer Beinprothese 31 gezeigt, die einen der Fig. 2 entsprechenden Schichtenaufbau aufweist. Am oberen Ende der Beinprothese 31 befindet sich eine Aufnahmeöffnung 33 für den Beinstumpf. Diese Aufnahmeöffnung 33 ist mit Ventilationsschichtmaterial 3 ausgekleidet und gegebenenfalls mit der Innenauskleidung 5. Das Ventilationsschichtmaterial ist derart orientiert, daß die Längsluftleitkanäle 19 zum oberen freien Ende der Beinprothese 31 führen, während die Querluftleitkanäle 21 oder 23 zum Beinstumpf hin gerichtet sind.

Die Ventilationswirkung des Ventilationsschichtmaterials 3 wird dadurch erhöht, daß durch Beinbewegungen, insbesondere beim Gehen, eine Pumpwirkung auf das Ventilationsschichtmaterial 3 ausgeübt wird. Durch diese Bewegung kann auch eine Pumpe, z.B. nach Art eines Blasebalges betätigt werden, mittels welcher Luft in das Ventilationsschichtmaterial 3 gepumpt wird.

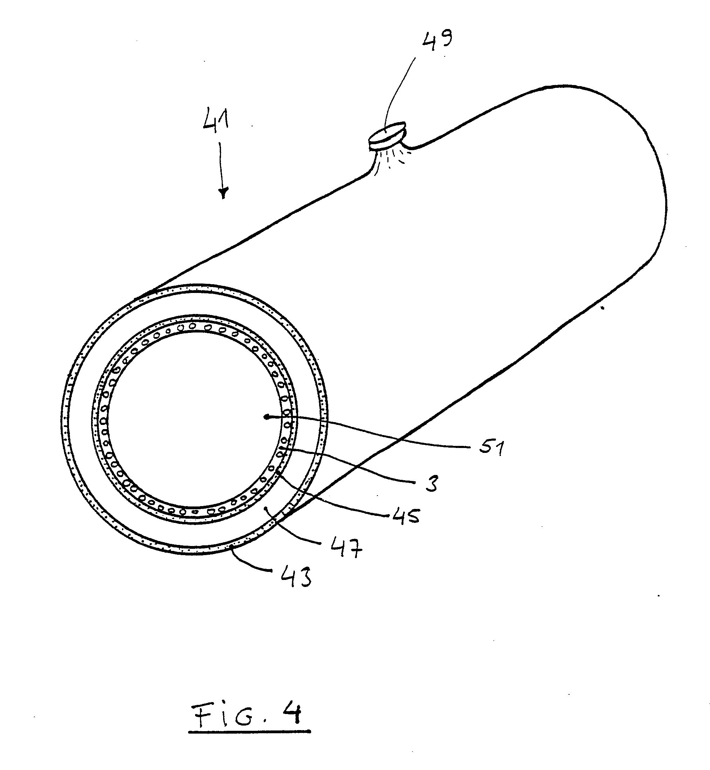

Fig. 4 zeigt in schematischer Darstellung ein Beispiel einer Luftschiene 41 in aufgeblasener und querschnittsmäßig durchgeschnittener Form. Zwischen einer Kunststoffaußenhaut 43 und einer Kunststoffinnenhaut 45 befindet sich eine Luftkammer 47. Mittels eines schematisch angedeuteten Aufblasventils 49 kann die Luftkammer 47 mit Luft gefüllt werden. Die innere Kunststoffhaut 45 ist mit Ventilationsschichtmaterial 3 ausgekleidet, derart, daß dessen Längsluftleitkanäle in Axialrichtung und dessen Querluftleitkanäle in Radialrichtung der röhrenförmigen Luftschiene 41 verlaufen. Innerhalb des Ventilationsschichtmaterials 3 befindet sich ein Innenraum 51 zur Aufnahme des zu schienenden Körperteils, beispielsweise eines Armes.

Zur Anwendung wird die röhrenförmige, beiden Endes offene Luftschiene 41 im nicht-aufgeblasenen Zustand über den zu schienenden Körperteil gezogen und über der zu schienenden Stelle plaziert. Dann wird mit Hilfe des Ventils 49 die Luftkammer 47 so weit aufgeblasen, bis die Innenwand der Luftschiene 41 dicht an dem zu schienenden Körperteil anliegt. Über das Ventilationsschichtmaterial 3 kann dann der Oberfläche des zu schienenden Körperteils Luft zur Kühlung und zur Abfuhr von Schwitzfeuchtigkeit zugeführt werden.

**Ansprüche**

1. Orthopädische Körperhilfsvorrichtung wie insbesondere Körpergliedprothese, Gipsschiene, Luftschiene oder ähnliches, die eine auf der Haut des Patienten aufliegende Aufstandsfläche aufweist,
dadurch **gekennzeichnet,**
daß mindestens die Aufstandsfläche der Körperhilfsvorrichtung mit flächigem, flexiblen Ventilationsschichtmaterial (3) ausgekleidet ist,
das Längsluftleitfähigkeit in Längserstreckungsrichtung und damit in Verbindung stehende Querluftleitfähigkeit zu mindestens einer seiner Oberflächen aufweist und das derart druckstabil ist, daß die Längsluftleitfähigkeit und
die damit in Verbindung stehende Querluftleitfähigkeit bei Normaldruckbelastungen, die beim normalen Gebrauch der mit dem Ventilationsschichtmaterial (3) versehenen Körperhilfsvorrichtung auftreten, aufrechterhalten bleiben, wobei die Längsluftleitfähigkeit zu einem offenen Ende der Körperhilfseinrichtung und die Querluftleitfähigkeit zur Hautoberfläche des Patienten gerichtet ist.

2. Orthopädische Körperhilfsvorrichtung nach Anspruch 1, dadurch gekennzeichnet, daß Querluftleitfähigkeit zu den beiden Oberflächen des Ventilationsmaterials besteht.

3. Orthopädische Körperhilfsvorrichtung nach Anspruch 1 oder 2, dadurch gekennzeichnet, daß die Längsluftleitfähigkeit durch Längsluftleitkanäle (19) und die Querluftleitfähigkeit durch Querluftleitkanäle (21, 23) bewirkt wird.

4. Orthopädische Körperhilfsvorrichtung nach einem der Ansprüche 1 bis 3, dadurch gekennzeichnet, daß das Ventilationsschichtmaterial eine porige Struktur mit sowohl in Querrichtung als auch in Längsrichtung offenen Poren aufweist.

5. Orthopädische Körperhilfsvorrichtung nach einem der Ansprüche 1 bis 3, dadurch gekennzeichnet, daß das Ventilationsschichtmaterial eine rippenartige Struktuur mit im wesentlichen parallelen Rippen auf mindestens einer Oberfläche aufweist.

6. Orthopädische Körperhilfsvorrichtung nach einem der Ansprüche 1 bis 3, dadurch gekennzeichnet, daß das Ventilationsschichtmaterial mindestens auf einer Oberfläche mit im wesentlichen parallelen Stegen (13 und/oder 17) versehen ist.

7. Orthopädische Körperhilfsvorrichtung nach einem der Ansprüche 1 bis 3, dadurch gekennzeichnet, daß das Ventilationsschichtmaterial eine genoppte Struktur mit Noppen auf mindestens einer Oberfläche aufweist.

8. Orthopädische Körperhilfsvorrichtung nach einem der Ansprüche 1 bis 3, dadurch gekennzeichnet, daß das Ventilationsmaterial als dreidimensionales Netzmaterial ausgebildet ist.

9. Orthopädische Körperhilfsvorrichtung nach einem der Ansprüche 1 bis 8, dadurch gekennzeichnet, daß von der Gesamtdicke des Ventilationsschichtmaterials (3) ein Anteil im Bereich von etwa 10 - 90 %, vorzugsweise von etwa 60 - 80 %, für den Luftleitfähigkeitsquerschnitt vorgesehen ist.

10. Orthopädische Körperhilfsvorrichtung nach einem der Ansprüche 1 bis 9, dadurch gekennzeichnet, daß das Ventilationsschichtmaterial (3) bei der Normal-Druckbelastung eine Mindestdicke von 1 mm aufweist.

11. Orthopädische Körperhilfsvorrichtung nach einem der vorausgehenden Ansprüche, dadurch gekennzeichnet, daß das Ventilationsschichtmaterial (3) auf der zur Haut des Patienten weisenden Seite mit einer Innenauskleidung versehen ist.

12. Orthopädische Körperhilfsvorrichtung nach einem der vorausgehenden Ansprüche, dadurch gekennzeichnet, daß eine durch Körperbewegung des Patienten oder elektrisch angetriebene Pumpe vorgesehen ist, die Ventilationsluft in das Ventilationsschichtmaterial (3) pumpt.

13. Orthopädische Körperhilfsvorrichtung nach Anspruch 12, in Form einer Beinprothese, dadurch gekennzeichnet, daß die Pumpe durch die Kniebewegung angetrieben wird.

FIG. 1

FIG. 2

INNENSEITE

AUSSENSEITE

5    3    1

FIG. 3

FIG. 4